(19) Europäisches Patentamt
European Patent Office
Office européen des brevets

(11) Publication number: **0 090 789**
**A1**

# (12) EUROPEAN PATENT APPLICATION

(21) Application number: **83870031.8**

(22) Date of filing: **24.03.83**

(51) Int. Cl.³: **C 07 H 21/00**

(30) Priority: **26.03.82 US 362576**

(43) Date of publication of application: **05.10.83**
**Bulletin 83/40**

(84) Designated Contracting States: **AT BE CH DE FR GB IT LI LU NL SE**

(71) Applicant: **MONSANTO COMPANY, Patent Department 800 North Lindbergh Boulevard, St. Louis, Missouri 63166 (US)**

(72) Inventor: **Adams, Steven Paul, 11025 Saturn Drive, Maryland Heights Missouri 63043 (US)**
Inventor: **Galluppi, Gerald Russell, 8 Hassam Court, Manchester Missouri 63011 (US)**

(74) Representative: **Lunt, John Cooper et al, Monsanto Europe S.A. Patent Department Avenue de Tervuren 270-272 Letter Box No 1, B-1150 Brussels (BE)**

## (54) Chemical DNA synthesis.

(57) A process for the production of oligonucleotides such as DNA is disclosed which comprises the phosphite triester method on inorganic solid support in which a hindered dialkylamino nucleoside phosphite having a total of 4 to 6 carbon atoms in the dialkyl group is employed as an intermediate reagent in the synthesis. The solid support preferably is controlled pore glass with a long spacer.

Background of the Invention

This invention relates to the chemical synthesis of oligonucleotides such as DNA.

DNA is a double-stranded, polymeric molecule comprised of a chain of deoxyribose residues linked by phosphodiester bonds. One of four bases, adenine (A), thymine (T), cytosine (C), or guanine (G) is connected to each sugar residue in the molecule. The DNA contains and transmits genetic information, and living cells use the information coded in DNA to synthesize proteins. This information is contained encoded by the sequence of the four bases.

With the advent of recombinant DNA technology in the past decade, a variety of biologically active proteins can now be synthesized by genetically engineered microorganisms which are not normally produced by such microorganisms. Hormones such as somatostatin, insulin, thymosin and human growth hormone are typical examples of such products. The importance of DNA synthesis as a fundamental feature of this genetic engineering for the construction of genes, probes, linkers, primers and the like is well-known.

Several general methods have been developed for the chemical synthesis of DNA. The first practical method - the so-called diester method - was developed primarily by Khorana and associates and is summarized in Science 203, 614-625 (1979). The basic step in this method is the joining of two appropriately protected deoxynucleotides to form dideoxynucleotide containing a phosphodiester bond. By removal of the protecting group from either end of the chain, the process can be repeated and the chain extended. A major disadvantage of this method is that it is very slow.

A more recently developed method, which has advantages in speed and yield, is the so-called triester method introduced by Letsinger and associates, J. Amer. Chem. Soc. 88, 5319 (1966), ibid 89, 4801 (1967). The principal difference between the diester method and the triester method is the presence in the latter of an extra protecting group on the phosphate atoms of the reactants and products. In the triester method the direct product of the condensation is a triester of phosphoric acid.

The basic triester method has been modified and improved by various investigators. The phosphite triester approach developed by Letsinger and co-workers constitutes one such improvement. J. Amer. Chem. Soc. 97, 3278-3279 (1975), ibid 98, 3655-3661 (1976). This approach involves the condensation reaction of an appropriately protected nucleoside, a bifunctional phosphitylating agent such as methoxydichlorophosphine and a second protected nucleoside.

According to another such improvement in the triester method, the solid-phase synthesis procedures for making polypeptides have been adapted to oligonucleotide synthesis. In these procedures, the initial nucleotide is attached to an inorganic solid support material such as silica and followed by the stepwise addition of nucleotides in any desired sequence. See, e.g., Blossey and Neckers, Eds., "Solid Phase Synthesis," Academic Press, New York, 1975.

Combinations of the phosphite triester procedure and the silica solid-phase procedure also have been developed independently by Ogilvie and associates, Tetrahedron Lett. (1980), p. 4159; Science 214, 270-274 (1981); and by Caruthers and associates, Tetrahedron Lett. 21, 719 (1980), ibid 22, 1859-1862 (1981) and J. Amer. Chem. Soc. 103, 3185-3191 (1981). The latter procedure also is described in Eur. Pat. Appl. 35,719. These two groups of investigators have adapted the phosphite triester silica solid-phase procedures to the automated synthesis of DNA as reported in their foregoing publications.

One of the main problems that still exists with the phosphite triester method is the instability of the reactive intermediate reagents such as the phosphochloridites, the dimethylaminophosphoramidites and the tetrazolides.

Further background information on the chemical synthesis of DNA can be had by reference to the review paper by Itakura and Riggs, Science 209, 1401-1405 (1980) and references cited therein.

## Description of the Invention

In accordance with the present invention, oligonucleotides such as DNA are synthesized by the phosphite triester method on an inorganic solid-phase support in which a bifunctional phosphitylating agent is used to provide a hindered dialkylamino nucleoside phosphite intermediate reagent and in which said dialkyl groups have a total of from 4 to 6 carbon atoms. The diethyl-, diisopropyl-, and methyldiisopropylamino nucleoside phosphites are illustrative of these hindered reagents.

The known dimethylamino nucleoside phosphite intermediate reagents described by Caruthers and co-workers, supra, have been demonstrated to be effective in the formation of internucleotide phosphate bonds. The reaction is fast and good yields can be obtained in reactions on solid supports. However, provision for increased reagent stability while maintaining a rapid condensation rate with solid supports is desirable and has been unexpectedly obtained with the larger molecule hindered derivatives of this invention compared to the smaller molecule dimethyl derivatives of Caruthers.

The intermediate dialkylamino nucleoside phosphite reagents employed in this invention can be represented by the following formula I:

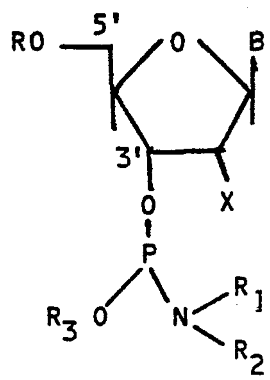

I

wherein B = protected purine or pyrimidine base,

R = protecting group,

$R_1 + R_2$ = alkyls in which the total number of carbon atoms is 4 to 6,

$R_3$ = alkyl having one to 6 carbon atoms, and

X = H or OR.

In the above formula, R can be any conventional protecting (blocking) group such as, for example, trityl, methoxytrityl, dimethoxytrityl, pivalylisobutyloxycarbonyl, t-butyl-dimethylsilyl and similar such protecting groups. X is H for DNA and OR for RNA oligonucleotides. In RNA, uracil replaces the thymine pyrimidine base of DNA.

It has been found that the hindered dialkylamino nucleoside phosphites used in the present invention exhibit substantially greater stability than the previously known dimethyl derivatives without loss of reactivity. The increased stability of these reagents makes them more amenable to use on automated gene synthesizers where stock solutions of reagents are desirably maintained for about 12 to 24 hours. Also, yields of DNA were obtained in the general range of about 50-100% higher using diethylamino nucleoside phosphites instead of the prior art dimethyl derivatives.

The hindered dialkylamino nucleoside phosphites are readily prepared by reaction of appropriately protected nucleosides or deoxynucleosides with excess dialkylaminoalkoxy halophosphines in which alkoxy preferably is methoxy and halo preferably is chloro or bromo but especially chloro. Illustrative of these phosphines are the diethyl-, diisopropyl-, and methylisopropylaminomethoxy chlorophosphines. Following hydrolytic workup, the desired phosphite product can be isolated by precipitation into cold hydrocarbon solvent such as cold hexane (e.g., at -70°C). Since these phosphite products are acid sensitive, they should be kept free of acidic impurities.

The dialkylaminoalkoxy halophosphines used in the foregoing reaction can be initially prepared by amination of alkoxydihalophosphine with about a two-fold molar excess of the corresponding dialkylamine accompanied by elimination of dialkylamino hydrohalic salt.

Further details on suitable methodology for making the initial dialkylaminomethoxychlorophosphines and then the dialkylamino nucleoside phosphites can be had by reference to Beaucage and Caruthers, Tetrahedron Lett. 22 (20), 1859-1862 (1981), and by substituting the larger molecule dialkylamines having a total of 4 to 6 carbon atoms for an equivalent amount of the dimethylamine reported in said publication.

Alternatively, the dialkylaminomethoxychlorophosphines can be prepared by heating mole equivalent amounts of dichloromethoxyphosphine and dialkylaminotrimethylsilane. Following evaporation of the byproduct, trimethylchlorosilane, the desired product can be distilled.

The aforesaid hindered dialkylamino nucleoside phosphite reagents can then be used in the phosphite triester synthesis of DNA on inorganic solid phase support materials in place of the dimethylamino nucleoside phosphite used for such synthesis in the prior art. In this synthesis, the principal novel feature of the present invention comprises condensing the 5'-OH group of a nucleoside or oligonucleotide covalently linked to an inorganic solid support through the 3'-O- linkage of said nucleoside or oligonucleotide with a dialkylamino nucleoside phosphite of formula I, *supra*, instead of the dimethylamino nucleoside phosphite.

In accordance with another aspect of the invention, it has been found that the use of controlled pore glass instead of ordinary silica gel of the prior art for the solid phase support provides further advantages in the DNA synthesis. For example, use of the controlled pore glass support in combination with the diethylamino nucleoside phosphites provides on the order of about 100% higher yields than obtained by use of the silica gel support with the corresponding dimethylamino nucleoside phosphite.

Controlled pore glass (CPG) is manufactured particularly as a support material for use in liquid chromatography and related processes. It is manufactured by Corning Glass Works, Corning, New York, and distributed by Pierce Chemical Company, Rockford, Illinois. CPG is produced from a borosilicate base material which has been heated to separate the borates and silicates. The borates are etched from the material, leaving a porous structure. Before etching, the material is ball milled and screened to a finished mesh size. Several sizes of CPG are available, e.g., 5-10, 37-74, 74-125, 125-177, and 177-840 microns. Several nominal pore sizes also are available, e.g., 40, 100, 250, 550, 1500 and 2500 angstroms. Any of these products can be used. The CPG is available either as plain, untreated glass, or covalently bonded to various compounds. A particularly desirable CPG is a CPG bonded to long chain alkylamine having a mesh size of 80/100 (125-177 microns) and a nominal pore diameter of 550 angstroms. This material is available from Pierce Chemical Company under Product Number 24875 and is reported to have the following general structure:

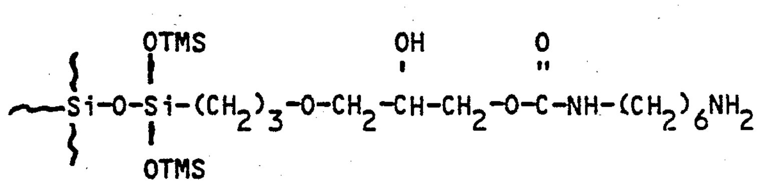

wherein TMS = trimethylsilyl.

The long chain terminal alkylamine arm in the above CPG product is reported to be about 20 angstroms long. This product is prepared by Glycophase (glycerol) coating of the CPG followed by further derivitization with 1,6-hexanediamine to provide a long chain spacer arm. Spacers of still greater chain length can be made by derivitization with alkyldiamines having alkyl groups of greater length, e.g., 1,12-dodecane-diamine. It is preferred that the total chain length of the spacer counting outwardly from the silicon atom with the pendant OTMS be such as to have at least 15 atoms in the chain, said atoms comprising generally carbon, oxygen and nitrogen. Use of 15-30 such atoms is eminently suitable.

Attachment of nucleoside to the solid support for use in the phosphite triester synthesis of DNA is conveniently carried out by conventional means. This attachment can be made by joining the derivitized solid support through amide or ester formation to the free carboxy group of nucleoside which has been modified to contain the half ester of a dicarboxylic acid, e.g., succinic or adipic acid. The chain length of the dicarboxylic acid can contribute to the total chain length of the spacer arm between the solid support and the nucleoside. Thus, adipic acid would add 10 atoms to the spacer.

The following examples will further illustrate the invention although it will be understood that the invention is not limited to these detailed examples. In these examples, NMR means nuclear magnetic resonance and TLC means thin layer chromatography.

The diisopropyl- and methylisopropyl- aminomethoxy-chlorophosphines and the corresponding nucleoside phosphites prepared therefrom in the following examples are novel compounds.

EXAMPLE 1

Preparation of N-protected 2'-deoxynucleosides:

N-6-benzoyl-2'-deoxyadenosine (2a)

To a stirred suspension of 20.0 g (0.080 mole) of 2-deoxyadenosine 1a (monohydrate is first dried by evaporation with pyridine) in 200 ml of anhydrous pyridine 56 g (0.40 mole) of benzoyl chloride is added dropwise over 15-20 minutes. The reaction is stirred for 2-1/2 hours after which it is poured into 2.0 liters of 0.01 M triethylammonium bicarbonate (TEAB) solution and extracted with dichloromethane (3 x 300 ml). The organic layer is concentrated to 140 ml on a rotary evaporator and then diluted to 400 ml with methanol and cooled on ice. With vigorous stirring, 240 ml of cold 2 N sodium hydroxide solution (1:1 methanol/water) is added to the ice cooled solution and the reaction is continued for 12-15 minutes at which point sufficient Dowex® 50X8 ion exchange resin (pyridinium form) is added to neutralize the reaction. The resin is filtered and washed with methanol and the filtrate is evaporated. The resulting oily residue is taken up in 250 ml of warm water and shaken with ether (2 x 250 ml) which is removed by aspiration. Cooling gives white crystalline product which is filtered, washed with cold water and dried to afford 20.3 g (71%) of product 2a with MP 112-121°C. (lit. 113-115*). NMR: $\delta$(DMSO d-6) 11.1(bs, 1H), 8.75(s, 1H), 8.70(s, 1H), 8.1(dd, J = 2,7 Hz, 2H), 7.5(m, 3H), 6.5(t, J = Hz, 1H), 5.4(bs, 1H), 5.1(bs, 1H), 4.5(m, 1H), 3.9(m, 1H), 3.6(m, 2H), 2.8(m, 2H).

* Schaller et al., J. Amer. Chem. Soc. 85, 3821(1963).

N-4-benzoyl-2'-deoxycytidine (2b)

To a stirred suspension of 21.1 g (0.08 mole) of 2'-deoxycytosine·HCl 1b in 250 ml of pyridine containing 12.0 g of triethylamine 56 g (0.40 mole) of benzoyl chloride is added dropwise over 15 minutes. The reaction is stirred at room temperature for 2-1/2 hours and then poured into 2.0 liters of 0.01 M TEAB. Extraction with dichloromethane (3 x 300 ml) followed by evaporation affords a white solid which is dissolved in 500 ml of THF and added to a solution of 500 ml THF, 700 ml methanol and 300 ml water and cooled on ice to 3°. With vigorous stirring, 200 ml of ice cold 2 N sodium hydroxide solution (1:1 methanol/water) is added and stirring is continued for 10 minutes. After neutralization with Dowex® 50X8 ion exchange resin (pyridinium form) and filtering, the solution is concentrated to 300 ml. After shaking with ether (2 x 300 ml - removed by aspiration) the solution is cooled to afford 19.0 g (71%) of product 2b after filtration and drying. MP 180-184°C. (lit. 194*). NMR: δ (DMSO d-6) 8.4(d, J = 7Hz, 1H), 8.0(dd, J = 2,8 Hz, 2H), 7.5(m, 3H), 7.3(d, J = 7 Hz, 1H), 6.1(t, J = 6 Hz, 1H), 4.2(m, 1H), 3.8(m, 1H), 3.6(m, 2H), 2.2(m, 2H).

*Schaller, supra.

N-2-isobutyryl-2'-deoxyguanosine (2c)

To a suspension of 21 g (0.078 mole) of 2'-deoxyguanosine 1c in 300 ml of anhydrous pyridine stirred at $0^o$ in an ice bath, 82 g (0.78 mole) of isobutyryl chloride is added over 1-1/2 hours. The reaction is stirred for an additional 1 hour at $0^o$ and then poured into 1.2 liter of 0.01 M TEAB and extracted with dichloromethane (3 x 200 ml). The organic layer is then back-extracted with 10% sodium carbonate solution, dried (sodium sulfate), and concentrated to 300 ml. With cooling on ice, 300 ml of cold methanol is added followed by 600 ml of 2 N sodium hydroxide solution (1:1 methanol/water). The reaction is stirred at $0^o$ for 25 minutes and neutralized with Dowex® 50X8 ion exchange resin (pyridinium form). The resin is filtered, washed with methanol and the filtrate is evaporated to a white solid which is redissolved in 250 ml of boiling water. Cooling overnight affords 20 g (75%) of crystalline product 2c after filtering and drying. MP > $300^oC$. NMR: $\delta$ (DMSO d-6) 12.1(bs, 1H), 11.6(bs, 1H), 8.2(s, 1H), 6.2(t, J = 7 Hz, 1H), 5.3(d, J = 5 Hz, 1H), 4.9(t, J = 6 Hz, 1H), 4.4(bm, 1H), 3.8(bm, 1H), 3.5(bt, J ~ 6 Hz, 2H), 2.7(q, J = 6 Hz, 1H), 2.3(bm, 2H), 1.1(d, J = 6 Hz, 6H).

## EXAMPLE 2

Preparation of 5'-O-(4,4-dimethoxytrityl) - protected nucleosides:

5'-O-(4,4'-dimethoxytrityl)-N-6-benzoyl-2'-deoxyadenosine (3a)

A solution of 36.9 g (0.104 mole) of N-benzoyldeoxyadenosine 2a and 38.8 g (0.115 mole) of dimethoxytrityl chloride in 300 ml of anhydrous pyridine is stirred at room temperature for 3 hours. Methanol (2-3 ml) is added and the solvent is removed in vacuo. The residue is taken up in ca. 100 ml of 0.01 M triethylammonium bicarbonate solution and extracted into dichloromethane which is dried (sodium sulfate) and evaporated. The residue is redissolved in 50 ml of 7:3 dichloromethane/acetone and is purified in five equal batches on a Waters Prep LC-500 using silica cartridges and eluting with 7:3 dichloromethane/acetone. The combined fractions containing product were evaporated, redissolved in 150 ml of chloroform and precipitated by dropwise addition to 3.0 liters of vigorously stirred pentane. The product was filtered and dried to afford 56.4 g (83%) of 3a *. M.P. 71-75°C. NMR: $\delta$ ($CDCl_3$) 9.3(bs, 1H), 8.7(s, 1H), 8.1(s, 1H), 8.0(dd, J = 2,8 Hz, 2H), 7.2-7.5(m, 12H), 6.7(B of AB, J = 7 Hz, 4H), 6.3(t, J = 6 Hz, 1H), 4.7(bm, 1H), 4.2(bm, 1H), 3.7(s, 6H), 3.4(m, 2H), 2.6(m, 2H).

*Schaller, *supra*.

5'-(4,4'-dimethoxytrityl)-N-4-benzoyl-2'-deoxycytidine (3b)

A solution of 26.6 g (0.08 mole) of N-benzoyl-2'-deoxycytosine 2b and 29.8 g (0.088 mole) of dimethoxytritylchloride in 250 ml of anhydrous pyridine is stirred at room temperature for 2 hours and the product is obtained after workup and chromatography (elution solvent - 55:45 dichloromethane/acetone) described above for the adenosine derivative. Yield, 38.8 g (77%). MP 113-126°C. (lit. 119*). NMR: $\delta(CDCl_3)$ 9.2(bs, 1H), 8.3(d, J = 8Hz, 1H), 7.8(dd, J = 7Hz, 2H), 7.2-7.5(m, 13H), 6.8(B of AB, J = 8 Hz, 4H), 6.3(t, J = 6 Hz, 1H), 4.5(bm, 1H), 4.2(bm, 1H), 3.7(s, 6H), 3.4(m, 2H), 2.8(m, 2H).

5'-(4,4'-dimethoxytrityl-N-2-isobutyryl-2'-deoxyguanosine (3c)

A solution of 25.3 g (0.075 mole) of N-isobutyryl-deoxyguanosine 2c and 280 g (0.083 mole) of dimethoxytritylchloride in 250 ml of anhydrous pyridine is stirred for 2 hours at room temperature and then worked up and isolated as in the case of the adenosine derivative (elution solvent - 55:45 dichloromethane/acetone) to afford 32 g (67%) of product. MP 136-142°C. NMR: $\delta(CDCl_3)$ 7.8(s, 1H), 7.1-7.3(m, 9H), 6.7(B of AB, J = 8 Hz, 4H), 6.1(t, J = 6 Hz, 1H), 4.7(bm, 1H), 4.2(bm, 1H), 3.7(s, 6H), 3.3(m, 2H), 2.4-2.8(m, 3H), 1.2(d, J = 6 Hz, 6H).

*Schaller, supra.

5'-(4,4'-dimethoxytrityl)thymidine (3d)

A solution of 24.2 g (0.10 mole) of thymidine 1d and 37.3 g (0.011 mole) of dimethoxytritylchloride in 300 ml of pyridine is stirred at room temperature for 4 hours. The solvent is evaporated in vacuo and the gummy product is dissolved in dichloromethane and extracted with 0.05 M triethylammonium bicarbonate solution (x2). The organic layer is dried (sodium sulfate) and evaporated and the resulting foamy residue is dissolved in 600 ml of boiling benzene. A crystalline product forms overnight which is filtered and boiled in 200 ml of benzene. The cooled mixture is filtered and dried to afford 50.6 g (93%) of product 3d. MP 119-123°C. (lit. 116-118*). NMR: $\delta$($CDCl_3$) 9.5(s, 1H), 7.5(s, 1H), 7.2-7.4(m, 9H), 6.8(B of AB, J = 8Hz, 4H), 6.4(t, J = 6 Hz, 1H), 4.5(m, 1H), 4.0(m, 1H), 3.7(s, 6H), 3.3(m, 2H), 2.3(m, 2H), 1.3(s, 3H).

EXAMPLE 3

3'-Succinyl Esters of 5'-dimethoxytrityl nucleosides (4)

A solution of 2.5 mmoles of 5'-(4,4'-dimethoxytrityl) nucleoside 3, 0.31 g (2.5 mmoles) of 4-dimethylaminopyridine and 0.25 g (2.5 mmoles) of succinic anhydride in 5 ml of pyridine is stirred at room temperature for 24 hours. Water (1 ml) is added and the solution evaporated. Toluene (10 ml) is added and evaporated and the residue is dissolved in dichloromethane which is extracted with ice cold 10% citric acid solution (x2), dried (sodium sulfate) and concentrated to a volume of 10 ml. A quantitative yield of product is obtained by precipitation into 150 ml of 1:1 pentane/ether followed by drying under high vacuum. The last traces of pentane are removed only with great difficulty as revealed by NMR, however, TLC (10 % water/acetonitrile) reveals a homogeneous product. This procedure was carried out on nucleosides 3a, 3b, 3c and 3d.

*Schaller, supra.

## EXAMPLE 4

Preparation of silica support using published method*

A suspension of 25 g of silica gel (Fractosil® 200-stored for 24 hours over a saturated lithium bromide solution) in 250 ml of toluene containing 15 ml of 3-aminopropyl triethoxysilane is shaken for 12 hours at room temperature and it is then heated at reflux for 18 hours. The silica is isolated by filtration and then washed with toluene, methanol and 1:1 methanol/water. After shaking overnight in 1:1 methanol/water, the silica is washed with methanol and ether and dried. The silica is then suspended in 50 ml of anhydrous pyridine and 15 ml of trimethylchlorosilane is added and the mixture is shaken overnight. The silica is isolated by filtration, washed with methanol and ether and dried, first in air, then in vacuo, and used below in Example 5.

## EXAMPLE 5

Preparation of nucleoside functionalized silica

A solution of 1.0 mmole of 3'-succinoyl-5'dimethoxytrityl nucleoside, 220 mg (10 mmoles) of dicylohexylcarbodiimide and 140 mg (1.0 mmole) of p-nitrophenol in 3 ml of anhydrous dioxane containing 0.3 ml of pyridine is stirred at room temperature for 2 hours. The urea precipitate is removed by centrifugation and the supernatant is transferred to a suspension of 2.5 g of aminopropylsilica in 7 ml of DMF containing 1 ml of triethylamine. This mixture is shaken for 2 hours. The silica is isolated by centrifugation, washed with DMF (x4), THF (x3), methanol (x3) and ether (x3). After drying, 5 ml of pyridine and 2 ml of acetic anhydride are added to the silica, and the reaction is allowed to proceed with occasional shaking for 30 minutes after which it is washed with methanol (x5) and ether (x2) and then pumped dry. Functionalized silicas obtained in this manner reproducibly have loadings of 80-110 μmoles of nucleoside per gram of silica as determined by quantitation at 497 nm in the visible spectrum of trityl cation released by acid. This functionalized silica was used as a solid support in Examples 8 and 12, below.

*Matteucci and Caruthers, J. Am. Chem. Soc. 103, 3185 (1981)

EXAMPLE 6

Preparation of dialkylaminomethoxychlorophosphines

Dialkylamine (2 moles) is added dropwise over 1 hour to a solution of 133 g (1 mole) of methoxydichlorophosphine* in 400 ml of ether stirring mechanically at -10°. The reaction is stirred additionally for 1 hour and the mixture is then filtered, the salt washed well with ether and the ether distilled at atmospheric pressure. Distillation (x2) provides product.

| | | | |
|---|---|---|---|
| Dimethylamino: | Bp 40-42° (13 mm) | NMR: | $^{31}P\ \delta$ 179.5 |
| Diethylamino: | Bp 87-89° (18 mm) | NMR: | $^{31}P\ \delta$ 179.3 |
| Diisopropylamino: | Bp 83-86° (12 mm) | NMR: | $^{31}P\ \delta$ 180.8 |
| Isopropylmethylamino: | Bp 62-64° (12 mm) | NMR: | $^{31}P\ \delta$ 176.8 |

The latter two products are novel products.

EXAMPLE 7

Preparation of N,N-dialkyl-methylnucleosidylphosphoramidites (6,7,8,9)

To a solution of 5 mmoles of protected nucleoside 3 in 10 ml of dry acid-free THF stirred at room temperature under a dry nitrogen atmosphere is added 4.3 ml (25 mmoles) of diisopropylethylamine followed by the dropwise addition over 1-2 minutes of 10-15 mmoles of either dimethylaminomethoxychlorophosphine (1.3 ml), diethylaminomethoxychlorophosphine (1.5 ml), diisopropylmethoxychlorophosphine (1.5 ml) or methylisopropylmethoxychlorophosphine (1.5 ml). The reaction temperature rises to ~40°. After

*See Martin et al., J. Am. Chem. Soc. 72, 4584 (1950)

stirring at room temperature for 15 minutes the reaction is diluted with 50 ml of acid free ethyl acetate and extracted with 10% sodium carbonate solution (x4). The organic fraction is dried (sodium sulfate), evaporated in vacuo, and then redissolved in 10 ml of acid free ethyl acetate. Product is obtained by precipitation into 300 ml of vigorously stirring hexane maintained at $-70^{o}$ followed by filtration and washing with $-70^{o}$ hexane (300 ml). While still cold, the filter, with the product left untouched, is transferred to a vacuum dessicator and dried under high vacuum for 24 hours. Yields typically exceed 90% with purities ascertained by $^{31}P$ nmr of the product and by tlc (1:1 ethylacetate/THF) of the product oxidized with 1% t-butylhydroperoxide in methanol. The results using protected nucleosides 3 a, b, c and d are summarized in Table 1.

Table 1

| Base | Product* | $\delta\ ^{31}P\ (CH_2Cl_2)$ | Purity |
|---|---|---|---|
| A | 6a | 147.1, 147.0 | 85-90%** |
| | 7a | 148.8, 148.6 | > 95%‡ |
| | 8a | 148.2, 148.0 | > 95% |
| C | 6b | 147.8, 146.8 | 80-85% |
| | 7b | 149.4, 149.1 | 92% |
| | 8b | 148.3, 148.0 | > 95% |
| G | 6c | 146.5, 146.3 | 85-90% |
| | 7c | 148.0, 148.2 | > 95% |
| | 8c | 148.4, 148.1 | > 95% |
| T | 6d | 147.3, 146.5 | 85-90% |
| | 7d | 148.7, 148.1 | 95% |
| | 8d | 148.1, 147.9 | > 95%' |
| | 9d | 147.1, 146.7 | > 92% |

* Nucleoside phosphite analogs

6 = dimethyl analog

7 = diethyl analog

8 = diisopropyl analog }

9 = isopropylmethyl analog } These are novel products

**Product free of starting nucleoside but contaminated with 10-15% of hydrolyzed material whose resonances appear at 8-20 ppm. Additionally, small amounts of hydrolyzed chlorophosphite appear at 17 ppm.

‡ Product free of starting nucleoside and of hydrolyzed product but 3-5% of hydrolyzed chlorophosphite persists ($\delta$ = 15 ppm).

EXAMPLE 8

DNA Synthesis Cycle Per Reaction Scheme 1

The synthesis of DNA on the solid support (Ⓢ in Scheme 1) described above is conducted most expeditiously by using a 15 ml sintered glass funnel of medium porosity with a Teflon® stopcock in the stem. By placing the funnel through a rubber stopper in a filtering flask, the system can be connected to a vacuum to aid in removal of solvents at each step. Depending on the amount of DNA desired, 50-100 mg (5-10 $\mu$moles of nucleoside) of nucleoside-silica is placed in the funnel and subjected to the sequence defined in Table 2. At each step the funnel is agitated gently to mix the contents. Detritylation of the nucleoside silica 5 and each subsequent trityl protected residue is effected rapidly with saturated $ZnBr_2$ in nitromethane containing 5% methanol to yield product 10 (Scheme 1); however, to insure complete deprotection, steps 1-3 (Table 2) are repeated until no more orange color is released upon $ZnBr_2$ treatment. After washing with methanol, THF and acetonitrile, the funnel is capped with a closure assembly having an injection port at the top closed with a rubber serum stopper and having a sidearm inlet port for provision of an inert gas blanket over the funnel contents. Then, under a dry argon atmosphere, 5 ml of acetonitrile freshly distilled from calcium hydride is added by syringe through the serum stoppered injection port. The solvent is vacuum filtered without allowing the silica to become completely dry. Nucleoside phosphite (45-90 mg, 10-20 equivalents) in 0.25 ml of dry acetonitrile is added by syringe, followed by injection of sublimed tetrazole (15-30 mg, 10-20 equivalents) in 0.5-0.75 ml of dry acetonitrile. The mixture is

agitated occasionally for 10 minutes after which it is filtered and submitted to a hydrolytic wash to give product 11 (Scheme 1). Oxidation to phosphate is then effected with $I_2$ providing product 12 (Scheme 1). Following a wash sequence with acetonitrile and with THF, acetic anhydride is added in the presence of dimethylaminopyridine to cap, and render unreactive, any 5' hydroxyl groups that did not react in the condensation step. Following a wash with methanol and with nitromethane, the sample is ready for another round of synthesis. One cycle of events requires a total of ~25 minutes and as many as six reaction vessels have been manipulated at one time. The condensation efficiencies can be roughly approximated by following spectrophotometrically the absorbance of each detritylation step. The amount of phosphite necessary for each condensation is variable and depends on the humidity above the reaction. Under carefully controlled conditions, 10 equivalents are usually sufficient for 90-95% yield.

Once the desired sequence has been achieved and the last nucleotide has been added and detritylated, the DNA is deprotected by removing the phosphate methyl groups using 2.0 ml of 1:2:2 thiophenol/triethylamine/dioxane for 45 minutes at room temperature. The silica is then suspended in 2.0 ml of conc. ammonium hydroxide for 2-1/2 hours which removes the DNA from the support. Heating the supernatant at $50^{o}$ for 20 hours removes all of the base protecting groups. The resulting solution is then chilled and carefully evaporated in vacuo to afford crude DNA which is purified in Example 9, below.

Reaction Scheme 1

The DNA synthesis cycle

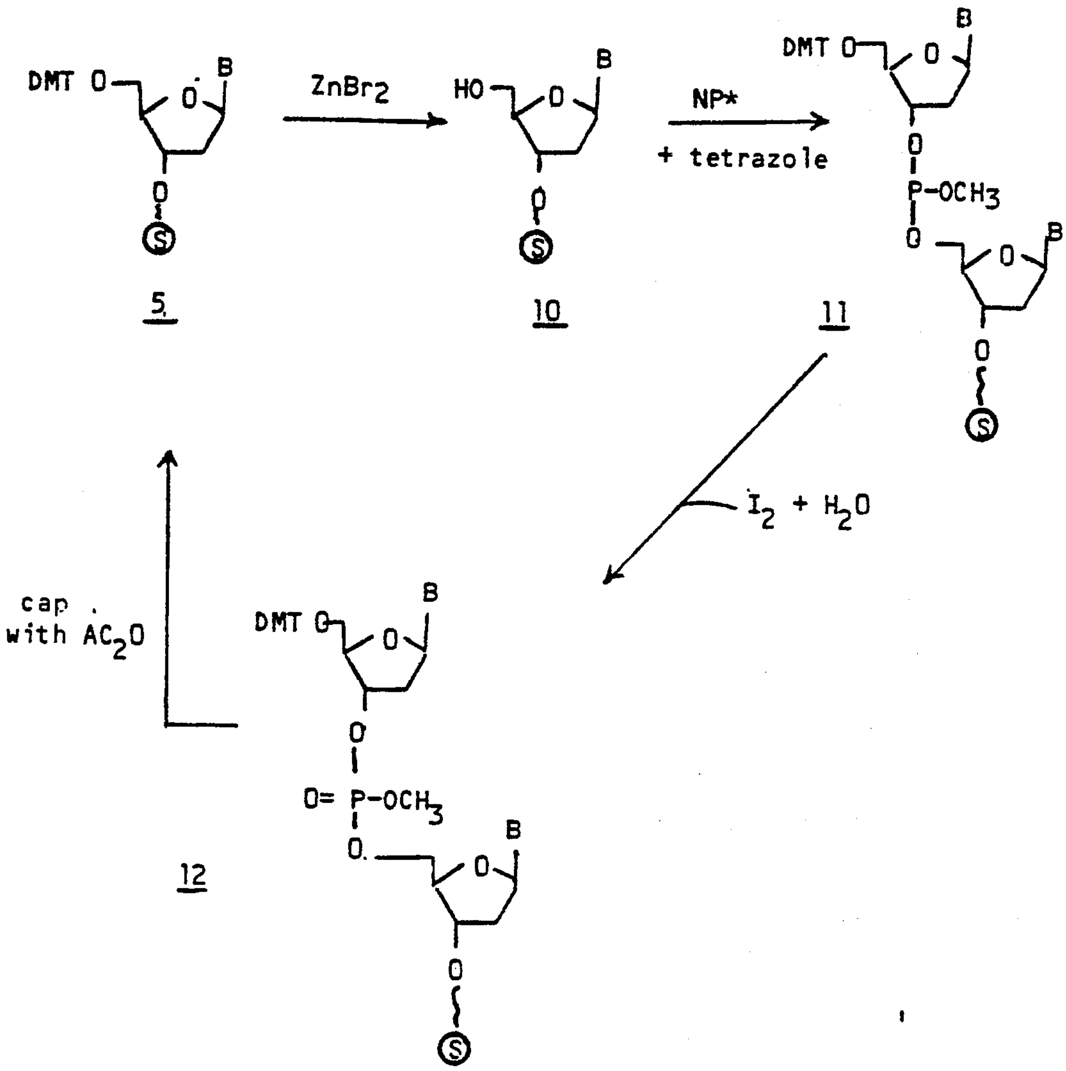


5

10

11

12

* Nucleoside phosphite
From Example 7

Table 2

DNA Synthesis Cycle
(25 minutes/cycle)

| Reagent or Solvent | Volume | Time |
| --- | --- | --- |
| 1. sat. $ZnBr_2$/5% methanol/nitromethane | 2.0 ml | 5 min |
| 2. methanol wash | 2 ml | |
| 3. nitromethane wash | 2 ml | |
| 4. repeat 1-3 until no more orange color is released. | | |
| 5. methanol wash | 2 ml | |
| 6. acetonitrile wash | 5 ml | |
| 7. serum stopper funnel/argon | | |
| 8. dry acetonitrile wash | 2x2 ml | |
| 9. 10-20 eq phosphite<br>+ 10-20 eq tetrazole | 0.24 ml<br>0.50 ml | 4 min |
| 10. 1:2:2 2,6-lutidine/water/THF | 5 ml | 1 min |
| 11. 0.2 M $I_2$/2,6-lutidine/water/THF | 5 ml | |
| 12. acetonitrile wash | 5 ml | |
| 13. THF wash | 5 ml | |
| 14. 0.5 M dimethylaminopyridine/THF<br>+ 1:1 acetic anhydride/2,6-lutidine | 1 ml<br>0.5 ml | 2 min |
| 15. methanol wash | 5 ml | |
| 16. nitromethane wash | 5 ml | |

eq = equivalents

An improved and preferred method of detritylating the 5'-hydroxyl protecting group from the support and each succeeding nucleoside (steps 1-4) in the growing chain comprises a two minute treatment with 2% (weight/volume) of dichloroacetic acid in dichloromethane, followed by washing as in steps 5 and 6, above. Other protic acids having a pK of from about 1.2 to about 1.8, and preferably about 1.5, can be used in place of dichloroacetic acid. See Example 13, below, in which this improved detritylating step was used in the DNA synthesis.

## EXAMPLE 9

DNA Purification

The crude DNA residue is resuspended in 250-300 μl G-50 column buffer (25 mM Tris-HCl, pH = 7.6 + 100 mM NaCl) and loaded onto a 10 ml G-50-40 Sephadex® column equilibrated with the same buffer. The sample is eluted and 1 ml fractions are collected. After reading the optical density at 260 nm for each fraction, the first four column fractions having absorbing material are tested for the desired DNA product by polyacrylamide gel electrophoresis: An aliquot of the column fraction (approximately 1 O.D. unit, usually 40-60 μl) is cooled to $0^{o}$. Five volumes absolute ethanol is added, and the sample is placed on dry ice for 20-30 min. After centrifugation (3 min in an Eppendorf 5412), the supernatant is decanted and residual solvent is removed in vacuo. The residue is resuspended in 10 ul formamide containing 0.01% bromphenol blue and 0.01% xylene cyanol. After heating for 1 min at 100°C, the samples are chilled on ice and loaded onto a 20% polyacrylamide gel (140 x 160 x 1.5 mm, 8 mm wide slots) containing 7 M urea. Samples are electrophoresed toward the anode at 300 V. On 20% acrylamide, bromphenol blue co-migrates with a DNA 7-mer while xylene cyanol, runs with a 25-mer (omit the dye if it will obscure the desired DNA). DNA is visualized by short wave UV shadowing on a Brinkman Polygram CEL 300 polyethyleneimine thin layer chromatography (TLC) plate.

Preparative scale electrophoresis is performed as follows: An aliquot of the G-50 column fraction containing 3-5 O.D. units of DNA is precipitated with 5 volumes of ethanol as above. After centrifugation (3 min in an Eppendorf 5412 or 45 min in a Beckman J2-21 at 10 k rpm) the supernatant is decanted and residual ethanol is removed in vacuo. The dried DNA is resuspended in 40 μl formamide plus appropriate tracker dye(s). Following treatment at 100° for 1 min, the sample is loaded onto a 20% acrylamide gel (140 x 160 x 1.5 mm, 35 mm wide slots). After electrophoresis, the DNA is visualized by UV shadowing. The plug of acrylamide containing the desired DNA band is cut out and extruded through a 3 ml B-D disposable syringe (no needle) into a 1.5 ml Eppendorf tube. Column buffer + 0.1% SDS is added (~1.0 ml), and the sample is extracted overnight. Acrylamide pieces are removed by filtration through glass wool. More column buffer (1.0 ml) is used to wash the acrylamide. The filtrate is twice extracted with 2 ml n-butyl alcohol, and the DNA is ethanol precipated as above. The pure product is resuspended in 1 ml G-50 buffer, and the O.D. at 260 nm is determined (1 O.D. unit = 35 γ DNA). (min = minutes)
(B-D = Becton Dickinson)

EXAMPLE 10

The stability of the dialkylamino nucleoside phosphites prepared in the above examples was evaluated by testing their half-life behavior in acetonitrile. The results are set forth in Table 3 which shows the stability sequence:

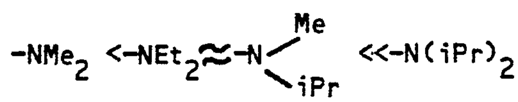

Table 3

Stability of Nucleoside Phosphites in Acetonitrile at Room Temperature

| Nucleoside Phosphite | $T_{1/2}$ (hrs)[3] | | | |
|---|---|---|---|---|
| | $NMe_2$ | $NEt_2$ | $N(iPr)_2$ | 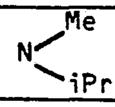 |
| 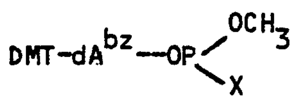 | | 45 | > 6 days | |
| DMT-dC$^{bz}$-- " | 8 | 30 | >6 days[2] | |
| DMT-dG$^{ib}$-- " | | 48[1] | >6 days | |
| DMT-dT -- " | | 6 days | >6 days | >6 days |
| DMT-dC$^{tol}$-- " | | >5 days | | |

1. Value is an estimate as decomposition product precipitated.
2. There is no change in the spectrum after 64 hours.
3. Half lives were determined by following the disappearance of phosphite peaks at 146-148 ppm and appearance of decomposition products at 0-20 ppm in the $^{31}P$ nmr spectrum. Solutions were ca. 0.05 M in phosphite.

X = Dialkylamino group
Me = Methyl
Et = Ethyl
iPr = Isopropyl
bz = Benzoyl
tol = o-Toluyl

## EXAMPLE 11

### Preparation of controlled pore glass (CPG) support and functionalizing with nucleoside

A suspension of 2.0 g of CPG, Pierce Chemical Company, Product Number 24875, 0.425 g of DMT-dN-succinyl ester (compound 4, Example 3), 0.600 g of 1-ethyl-3-(3-dimethylamino-propyl)carbodiimide hydrochloride (Sigma Chemical Company), and 0.030 g of 4-dimethylaminopyridine in 8 ml of dimethylformamide (DMF) and 2 ml of pyridine was shaken gently for 18 hours, after which it was filtered and washed with DMF and tetrahydrofuran (THF). The support was then resuspended in 4 ml of pyridine containing 2 ml of acetic anhydride and shaken for 2 hours. After filtering and washing with THF, the support was dried in vacuo. Functionalized CPG obtained in this manner reproducibly had loadings of about 40 $\mu$ moles of nucleoside per gram of CPG as determined by quantitation at 498 nm in the visible spectrum of trityl cation released by acid. This functionalized CPG was used as the solid CPG support in Example 12, below.

## EXAMPLE 12

### DNA Synthesis

A large number of DNA syntheses using the method described hereinbefore have reproducibly shown the superiority of the hindered dialkylamino nucleoside phosphites and the CPG solid support when compared to the dimethylamino nucleoside phosphites and the silica support. The following parts illustrate these improvements.

A. Attempts to prepare the following 23-mer failed to give product in several runs using dimethylamino nucleoside phosphites on silica support; however, when the corresponding diethylamino nucleoside phosphite on silica support was used, a 5% yield of product was obtained which was isolated, purified and shown to be biologically active.

$^{5'}$CCGGAGATCTGCAGATTATTTGG$^{3'}$

B. The following 14-mers which are probes for isolating specific m-RNA's were synthesized using diethylamino nucleoside phosphites on silica (I) and CPG (II) supports:

```
5'ACATTCCAACTCAT3'        (I)
   G     G

5'ACATTCCAAGACAT3'        (II)
   G     G
         T
         C
```

The overall yield of I was 18% whereas the overall yield of II was 35%.

In addition to improved yields with CPG (as assayed by the final detritylation), the DNA in II contained more of the desired product with fewer failure sequences as determined by gel electrophoresis on samples labeled with $^{32}P$.

C. In another DNA syntheses, two 17-mers, primers for Sanger's dideoxy sequencing in M-13, the reverse primer was synthesized on silica (III) and the forward primer was synthesized on CPG (IV) using diethylaminomethoxy nucleoside phosphite. See Sanger et al., PNAS USA, 74(n), 5463-5467 (1977).

$^{5'}$CAGGAAACAGCTATGAC$^{3'}$ (III)

$^{5'}$GTAAAACGACGGCCAGT$^{3'}$ (IV)

The production of IV on CPG was qualitatively higher than III and showed fewer failure sequences as by-products.

D. In the syntheses of the following 10-mer on CPG (V), diethyl- and diisopropyl nucleoside phosphites each provided yields of about 40% and gave DNA of equal quality. This demonstrates relatively equivalent effectiveness of the diethyl- and diisopropyl analogs.

$^{5'}$GGAATTCTTT$^{3'}$ (V)

EXAMPLE 13

In another DNA synthesis, the following 17-mer (VI) was prepared on CPG solid support substantially according to the method described in Example 12, above, except that dichloroacetic acid (pK 1.5) was used as the detritylating agent instead of $ZnBr_2$, as described at the end of Example 8, above. The total synthesis time was reduced by about one-third and the yield was increased by about 100% by this improved detritylation step.

$^{5'}$GTCATAGGTGTTTCCTG$^{3'}$ (VI)

Various other examples can be devised by the person skilled in the art after reading the foregoing disclosure without departing from the spirit and scope of the invention, and it is intended that all such examples be included within the scope of the apended claims.

WHAT IS CLAIMED IS:

1. A process for the production of oligonucleotides which comprises the step of condensing the 5'-OH group of a nucleoside or oligonucleo tide covalently linked to an inorganic solid support through the 3'-O- linkage of said nucleosideor oligonucleotide with a hindered dialkylamino nucleoside phosphite of the following formula

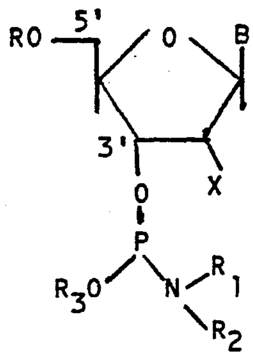

wherein B = protected purine or pyrimidine base,

R = protecting group,

$R_1$+ $R_2$ = alkyls in which the total number of carbon atoms is 4 to 6,

$R_3$ = alkyl having one to 6 carbon atoms, and

X = H or OR.

2. The process of Claim 1 in which the dialkylamino group is diethylamino.

3. The process of claim 1 in which the dialkylamino group is diisopropyl

4. The process of Claim 1 in which the dialkylamino group is methylisopropyl.

5. The process of Claim 1 in which R is a trityl protecting group.

6. The process of Claim 1 in which the inorganic solid support is silica.

7. The process of Claim 1 in which the inorganic solid support is controlled pore glass.

8. The process of Claim 1 in which the inorganic solid support is linked to nucleoside or oligonucleotide through a long chain spacer having at least 15 atoms in the chain.

9. The process of Claim 1 in which the dialkylamino group is diethyl and the inorganic solid support is controlled pore glass with a long chain spacer having at least 15 atoms in the chain.

10. The process of Claim 1 in which the dialkylamino group is diisopropyl and the inorganic solid support is controlled pore glass with a long chain spacer having at least 15 atoms in the chain.

11. The process of Claim 1 in which the dialkylamino group is methylisopropyl and the inorganic solid support is controlled pore glass with a long chain spacer having at least 15 atoms in the chain.

12. Diisopropylaminomethoxychlorophosphine.

13. Methylisopropylaminomethoxychlorophosphine.

14. Diisopropylamino nucleoside phosphite.

15. Methylisopropylamino nucleoside phosphite.

16. A process for the production of oligonucleotides which comprises the step of condensing the 5'-OH group of a nucleoside or oligonucleotide covalently linked to an inorganic solid support through the 3'-O- linkage of said nucleoside or oligonucleotide with a dialkylamino nucleoside phosphite of the following formula

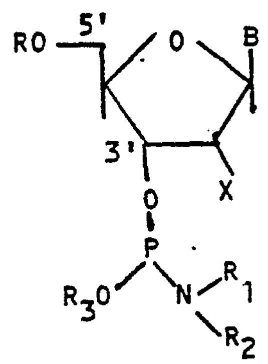

wherein B = protected purine or pyrimidine base,

R = protecting group,

$R_1$, $R_2$, and $R_3$= alkyls having from one to 6 carbon atoms,

X = H or OR,

and in which the inorganic solid support is linked to nucleoside or oligonucleoside through a long chain spacer having at least 15 atoms in the chain.

17. The process of Claim 1 in which the protecting group is a trityl group and in which detritylation of protected nucleoside residue is carried out by treatment with a protic acid having a pK of from about 1.2 to about 1.8.

18. The process of Claim 17 in which the protic acid is dichloroacetic acid having a pK of about 1.5.

0090789

Application number

European Patent Office

# EUROPEAN SEARCH REPORT

EP 83 87 0031

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | TETRAHEDRON LETTERS, vol. 22, no. 20, 1981, pages 1859-1862, Pergamon Press Ltd., GB S.L. BEAUCAGE et al.: "Deoxynucleoside phosphoramidites-a new class of key intermediates for deoxypolynucleotide synthesis" * Page 1859 * | 1 | C 07 H 21/00 |
| | --- | | |
| Y | EP-A-0 035 719 ((UNIVERSITY PATENTS INC.) * Pages 12-13 * | 1 | |
| | --- | | |
| P,X | EP-A-0 061 746 (UNIVERSITY PATENTS INC.) * Page 40 * | 1-18 | |
| | ----- | | TECHNICAL FIELDS SEARCHED (Int. Cl. 3) |
| | | | C 07 H 21/00 |

The present search report has been drawn up for all claims

| Place of search | Date of completion of the search | Examiner |
|---|---|---|
| THE HAGUE | 20-05-1983 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82